# EUROPEAN PATENT APPLICATION

(11) **EP 4 740 939 A1**
(43) Date of publication of application: **13.05.2026**
(21) Application number: 24211874.3
(22) Date of filing: 08.11.2024
(51) Int. Cl.: A61K 9/28

(54) **COATING SOLUTIONS WITH IMPROVED COVERING AND WHITENING EFFECT**

(71) Applicant: Merck Patent GmbH, 64293 Darmstadt (DE)
(72) Inventor: The designation of the inventor has not yet been filed
(74) Representative: Merck Patent Association

(57) **Abstract**

The invention relates to coating solutions comprising a polyol, a polymer, and a solvent, wherein the polyol is mannitol or xylitol and the polymer is an aqueous soluble polymer or a cellulose based polymer, and the use of said coating solutions in the coating of medicaments. The invention further relates to a method for coating a medicament and the use of mannitol in a coating solution for medicaments.

## Description

### Field of the invention

The invention relates to coating solutions with improved covering and whitening effects on the surface of a medicament.

### Technical background

Regulatory concerns regarding the use of titanium dioxide as a whitening pigment in coating formulations are on the rise, which makes it important to provide alternatives for formulation development.

Classical approaches for titanium dioxide replacement include the use of other pigments like zinc oxide, calcium phosphate, magnesium carbonate or calcium carbonate. Sugar-based formulations have also been developed in order to enhance appearance, taste, and stability of pharmaceutical and non-pharmaceutical products in solid dosage forms. However, sugar-based solutions present their own disadvantages and limitations, for example due to considerable dosage form size increase, higher caloric content, or the effects on blood sugar levels.

Recently, the replacement of sugar by sugar-free alternatives has been described in scientific literature. However, providing sugar-free alternatives with suitable whitening and covering effects remains a challenge.

The whitening effect of a coating solution is not a mere aesthetic or marketing aspect. Among the many complex factors involved in the success or failure of a drug therapy are behavioral elements such as patient compliance. Compliance is notably impacted by feelings of fear, anxiety, and mistrust in a patient. Medicaments that are coated with an intense, sharp white color have been shown to convey several characteristics that significantly contribute to patient compliance, such as safety, purity, or calm.

In practice, coating solutions are applied to medicaments by spraying. This process also presents challenges as it puts constraints on the potential compounds and their concentrations that might be selected for mixing in the coating solution.

### Objective problem to be solved

To address these challenges, alternative sugar-free coating solutions are required which show a satisfactory whitening effect, are chemically stable and comply with drug requirements.

### Summary of the invention

In one aspect, the invention relates to a coating solution comprising a polyol, a polymer, and a solvent, wherein the polyol is mannitol or xylitol and the polymer is an aqueous soluble polymer or a cellulose based polymer.

In a second aspect, the invention relates to the use of the coating solution according to the invention as a coating of a medicament.

In a third aspect, the invention relates to a method for coating a medicament, comprising the steps of a) mixing a solution comprising a polyol, a polymer, a plasticizer, and a solvent, b) spraying the solution of step a) on a medicament, and c) allowing the evaporation of the solvent, wherein the polyol is mannitol or xylitol and the polymer is an aqueous soluble polymer or a cellulose based polymer.

In a fourth aspect, the invention relates to the use of mannitol in a coating solution for a medicament.

In a fifth aspect, the invention relates to the use of mannitol in a coating solution or a coating solution comprising mannitol, preferably as described hereinafter, for imparting a white color to a medicament.

### Brief description of the figures

**Fig. 1** shows the results of the color measurement of different pigment containing films on the black color cards.
**Fig. 2** shows the results of the color measurements of mannitol containing films at different concentrations on the black color cards.
**Fig. 3** shows the results of the color measurements for mannitol coated tablets in comparison to calcium carbonate and titanium dioxide.

### Detailed description of the invention

In one aspect, the invention relates to a coating solution comprising a polyol, a polymer, and a solvent, wherein the polyol is mannitol or xylitol and the polymer is an aqueous soluble polymer or a cellulose based polymer.

The coating solution according to the invention provides a coating of a medicament. The coating solution according to the invention provides a strong whitening and covering effect on the surface of a medicament without requiring titanium dioxide or any other insoluble or poorly soluble pigments. The coating solution also provides a coating of a medicament with outstanding light reflection as well as the covering of undesired colors of the core of the dosage form. The strong covering effect on a medicament, in turn protects the core of the dosage form from environmental factors such as light and humidity, thus enhancing the stability and shelf-life of the active pharmaceutical ingredient. Similar effects can be achieved when using sorbitol as the polyol.

According to the invention, the medicament is preferably a solid dosage form such as a pill, a granule, a tablet or capsules. Most preferably, the medicament is a tablet.

In some embodiments, the coating solution further comprises a plasticizer. A plasticizer increases the pliability and flexibility of the coating film produced from the coating solution. The plasticizer is preferably a low molecular weight compound. In some embodiments, the polyol itself acts as plasticizer in the coating solution.

In some embodiments, the plasticizer is selected from the group comprising triethylcitrate, triacetin, glycerol, and polyethylene glycols of molecular weights between 600 and 6000 Da, preferably between 1000 and 3000 Da. Low molecular weight plasticizers are more effective at increasing the pliability and flexibility of the coating film resulting from the coating solution.

In some embodiments, the aqueous soluble polymer of the coating solution is selected from the group consisting of polyvinyl alcohol (PVA), polyvinyl pyrrolidone, methacrylic acid copolymers, and shellac; and/or the cellulose based polymer is selected from the group comprising methylcellulose, hydroxypropyl cellulose (HPC), hydroxypropyl methylcellulose (HPMC or hypromellose), hydroxyethyl cellulose (HEC), hydroxyethyl methyl cellulose (HEMC), ethylcellulose (EC), and cellulose acetate phthalate. The selection of polymer for a coating solution is a deciding aspect in the reduction of residual solvents, the level of which must be sufficiently low not to pose a safety and environmental concern. Aqueous soluble and cellulose-based polymers have been found to provide the best results in minimizing residual solvent in the coating solution.

In a preferred embodiment, the polyol is mannitol and the polymer is PVA. Mannitol increases the whitening and covering effect of the coating solution on the surface of a medicament. It is further advantageous because of its capacity to crystallize and form a fine coating film on the medicament surface during evaporation of the respective solvent after spraying. As is demonstrated in the examples below, the particular combination of mannitol as a polyol and PVA as a polymer results in optimal whitening and covering effects of the coating solution.

In another preferred embodiment, the coating solution comprises mannitol at a w/w% concentration between 10% and 30%, preferably between 20% and 30%. The advantages of mannitol as the polyol in the coating solution are enhanced when a coating solution of high mannitol concentration is provided, particularly in these concentration ranges.

In yet another preferred embodiment, the w/w% ratio of mannitol to PVA is between about 50:50 and about 15:85. As is demonstrated in the examples below, the whitening and covering effects of the coating solution are better within said w/w% ratios of mannitol to PVA.

The PVA is preferably selected between PVA 3-82, PVA 4-88, PVA 5-88, PVA 8-88, Preferred PVA is PVA 5-88. PVA 5-88 is a polyvinyl alcohol whose viscosity is approximately 5 mPas as a 4% solution in water at 20 °C and an approximate degree of hydrolysis of polyvinyl acetate groups of 88%. Most preferred is a PVA 5-88 having a particle size dv(0.10) between 30 and 120 µm, dv(0.50) between 180 and 260 µm and dv(0.90) between 300 and 700 µm.

In one embodiment, the coating solution comprises an anti-tacking agent. Preferably, the anti-tacking agent is selected from the group consisting of talcum, magnesium stearate, microcrystalline cellulose, glyceryl monostearate, stearic acid, kaolin and colloidal silicon dioxide. Anti-tacking agents are used in the pharmaceutical industry to prevent tackiness of the dosage forms during the manufacturing process and storage.

In one embodiment, the solvent is a water-based solvent, an alcohol, a ketone, an ester or a chlorinated hydrocarbon or a mix thereof. Preferably, the solvent is a water-based solvent.

As used herein, "water-based solvent" is any medium used to dissolve one or more other substances of a mixture, wherein water is a component of the medium, preferably the main component of the medium.

Water-based solvents or combinations thereof are advantageous due to the absence of potentially toxic solvents, whereas organic solvents require shorter drying times and are advantageous when formulating drug-loaded coatings.

In some embodiments, the color distribution values of the coating solution according to L*a* b* values of CIELAB color space are L* > 90, preferably L* > 95, -5 < a* < 10, preferably -2 < a* < 5, preferably -1 < a* < 1, more preferably a* = 0, and -5 < b* < 10, preferably -2 < b* < 5, preferably -1 < b* < 1, more preferably b* = 0, as measured by color card trials. In this case, values of CIELAB color space are used to indicate level of whiteness in color trials. As is demonstrated in the examples, the coating solution according to the invention is able to achieve values of L* close to 100 as well as a* and b* values tending to 0, which indicates a strong white color. The color distribution values of the coating solution are not directly measured from the solution but from the coating films formed on the color cards after application of the coating solution. Particularly important is the measurement of the black colored part of the color cards, which allows to better assess the covering effect of the coating solution. As demonstrated in the examples, the L*a*b* values of the coating solution described herein are achievable for the black colored part of the color cards, thus confirming the covering of a black surface.

In a second aspect of the invention, the coating solution as described above is used as a coating of a medicament. The use of the coating solution according to the invention provides a strong whitening effect without requiring titanium dioxide or any other insoluble or poorly soluble whitening pigments. Medicaments that are coated with an intense, sharp white color have been shown to convey several characteristics that significantly contribute to patient compliance, such as safety, purity, or calm. The use also provides a coating of a medicament with outstanding light reflection as well as the covering of undesired colors of the core of the dosage form. The strong covering effect on a medicament in turn protects the core of the dosage form from environmental factors such as light and humidity, thus enhancing the stability and shelf-life of the active pharmaceutical ingredient.

In a third aspect of the invention, a method is provided for coating a medicament, comprising the steps of a) mixing a solution comprising a polyol, a polymer, a solvent and optionally a plasticizer, b) spraying the solution of step a) on a medicament, and c) allowing the evaporation of the solvent, wherein the polyol is mannitol or xylitol and the polymer is an aqueous soluble polymer or a cellulose based polymer. By selecting mannitol or xylitol as a polyol and applying the mixed solution by spraying, the polyol crystallizes on the medicament surface during evaporation of the solvent and creates a fine white covering or coating film on the medicament that provides a strong whitening and covering effect. The coating film created by spraying and evaporation of the solution of step a) provides outstanding performance in terms of light reflection as well as the covering of undesired colors of the core of the dosage form.

In one embodiment, the mannitol is added during step a) at a w/w% concentration between 10% and 30%, preferably between 20% and 30%.

In some embodiments, the polyol crystallizes on the medicament surface during step c). Evaporation of the solvent increases the local polyol concentration, leading to crystallization of the polyol already during the spraying stage.

In some embodiments, the solvent is a water-based solvent, an alcohol, a ketone, an ester or a chlorinated hydrocarbon or a mix thereof. Preferably, the solvent is a water-based solvent.

In some embodiments, wherein a water-based solvent is used, a second solvent is added during step a) at a w/w% concentration of up to 30% of the water-based solvent in order to improve evaporation during step c). The mixing of different solvents can be useful in accelerating evaporation and thus the rate at which polyol concentration is increased, in order to achieve better spraying efficacy.

In a fourth aspect, the invention relates to the use of mannitol in a coating solution for a medicament, said coating solution being in particular as described above. The invention also relates to the use of mannitol for preparing a coating solution for a medicament, said coating solution being in particular as described above.

In a fifth aspect, the invention relates to the use of mannitol or a coating solution comprising mannitol, preferably as described above, for imparting a white color to the coating of a medicament.

### Examples

### Example 1: Film formation

### Color card trials with different whitening pigments

| Equipment | Manufacturer |
|---|---|
| Color card puller | Moeller, CI-K3-125-M |
| Colorimeter | Konica Minolta Spectrophotomer, CM-700d |
| Heating plate | Merck build |
| Mixer | Hauschild Engineering, SpeedMixer DAC 150 FVZ |
| Stirrer | IKA-Werke, Eurostar P1 / Janke&Kunkel IKV Werke, RW 20 DZM |
| Stirring plate | Heidolph, MR 3004 safety |
| Balance | Sartorius, MSU524S-100-DI |
| | Sartorius. M-Power AZ3102 |
| | Sartorius, LA6200S |

| Consumables | Manufacturer |
|---|---|
| Color cards | Eibunsya Insatsujyo Co Ltd.. 545, NAKANO, Midori-Ku, SAGAMIHARA-SHIKANAGAWA, 220-0207 JAPAN 000-0000 JAPAN |
| Mixing beakers | Hauschild Engineering, Becher PP30- 60 mL, 39231090 |

### Chemicals

| Chemicals | Manufactuer | Batch no. |
|---|---|---|
| EMPROVE^{®} ESSENTIAL Ph Eur, BP, USP, E170, FCC Calciumcarbonate | Merck KGaA | K53435269 150 |
| Calciumphosphate | Sigma Aldrich | SLBF8704V |
| Calciumsulfate | Sigma Aldrich | 1003444779 |
| Caolin | Merck KGaA | K51876540 217 |
| galenIQTM 721 (Isolmalt Ph. Eur., BP, USP-NF, JP) | BENEO-Palatinin GmbH | L1211948U1 |
| Maltitol | Thermo scientific | 10232829 |
| Nativ rice starch 1 | BENEO-Remy N.V. | 2120189540 |
| Nativ rice starch 2 | BENEO-Remy N.V. | 2120179530 |
| Parteck ^{®}COAT PVA | Merck KGaA | F2053117 |
| EMPROVE^{®} ESSENTIAL Ph Eur, BP, ChP, USP, E421 Parteck^{®} M 100 (Mannitol) | Merck KGaA | MP21039594 149 |
| EMPROVE^{®} ESSENTIAL Ph Eur, BP, USP, E171 Titanium(IV)dioxide | Merck KGaA | K48897505 751 |
| EMPROVE^{®} ESSENTIAL Ph Eur, BP, ChP, USP Zincoxid | Merck KGaA | K51288546 |

To prepare the solutions, the appropriate amount of Parteck^{®} Coat was stirred in at room temperature and 450 rpm [IKA-Werke, Eurostar o. Janke& Kunkel IKV Werke, RW 20 DZM]. Subsequently, the suspension was heated at a temperature of about 90 °C (set temperature: 105 °C), 450 rpm for 30 min [Heidolph, MR 3004 safety]. Afterwards, the solution was cooled down to room temperature while stirring at 450 rpm. The amount of distilled water that escaped due to the heating was finally made up.

### Preparation of the pigment suspensions:

Eight different whitening pigments were investigated for their ability to serve as a whitening pigment. The pigments used were: Calciumphosphate, Calciumsulfate, zinc oxide, Maltitol, Isomalt, Caolin, rice starch and Mannitol. The solid content of each pigment suspension was 20% wt. Each suspension was prepared with the 20% Parteck coat solution that had been prepared before.

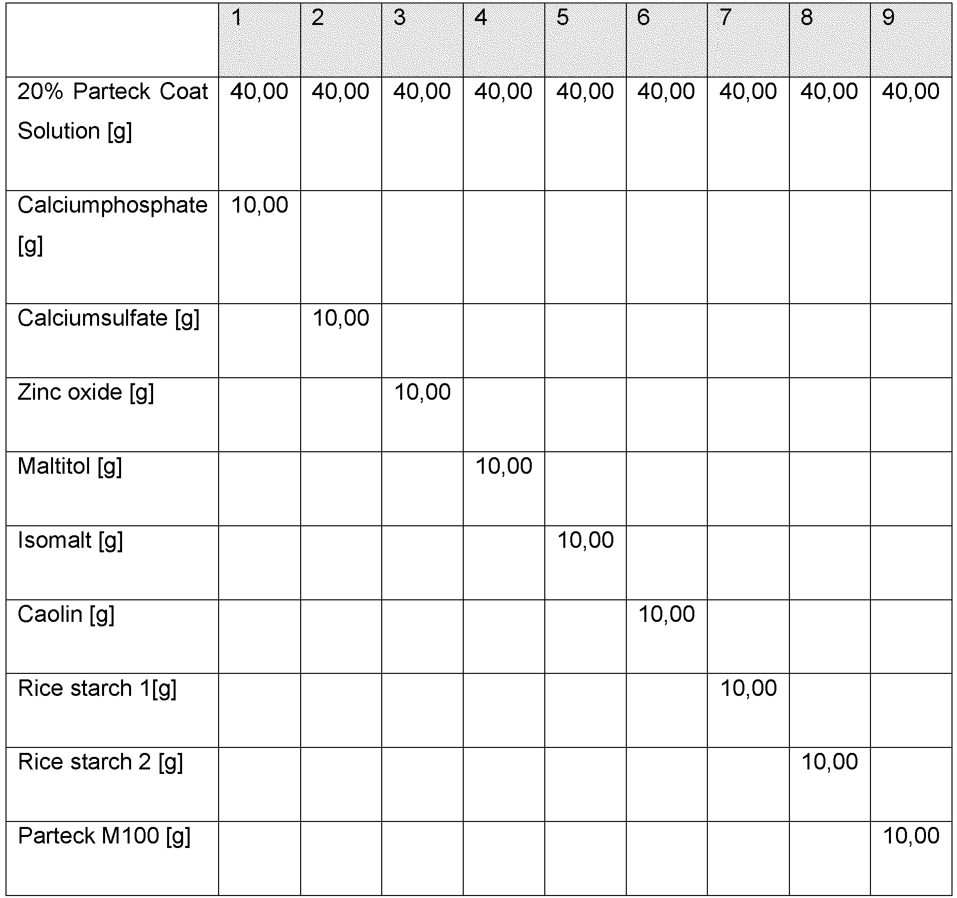

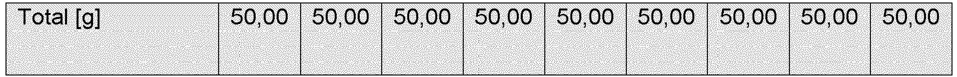

The needed amount of whitening pigment was weight in a Hauschild cup and then filled with the required amount of 20% Coating solution. The cup was then put in the Hauschild mixer for 2 minutes at 3300 rpm. The resulting suspension was applied directly to the color cards using a 500µm squeegee and color card puller. For each pigment, 3 coated color cards were prepared. The color cards coated in this way were then dried on a heating plate set at 50°C for 1 hour.

### Color card measurements

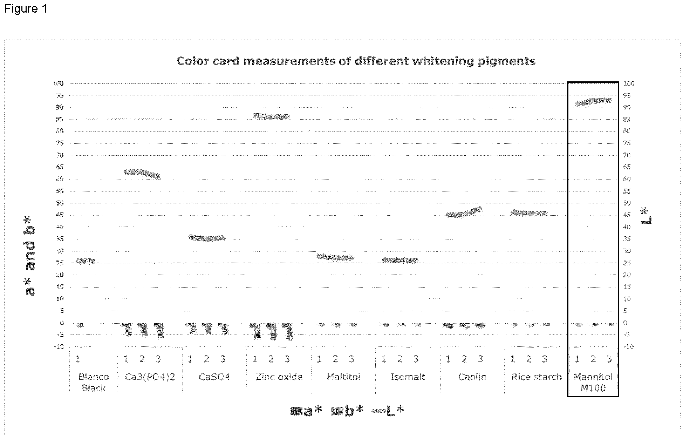

Color measurements were made using the Konica Minolta CM-700d spectrophotometer with a diskless MAV aperture. After the instrument was turned on, a zero calibration and a white calibration were first performed. The samples were each measured using the SCI and SCE methods. The black and the white section of a blanco color card were measured to generate reference values. For the actual measurement an area was defined for the color cards (4.5 cm up and down, 2.0 cm to the left and right of the center point) in which the measurements were conducted. Within this window, three points were measured in each of the white and black areas, and an average value was determined for the black and white sides using SpectraMagic NX software, which then described the color value for the black and white backgrounds of a color card. The results, given as L*a* b* values showed that Mannitol performed best in terms of whitening (see Fig. 1).

### Color card trials with different M100 concentrations

### Equipment:

▪ Color card puller: Moeller, CI-K3-125-M
▪ Highspeed mixer: Hauschild Engineering, SpeedMixerTM DAC 150 FVZ
▪ Colorimeter: Konica Minolta Spectrophotomer, CM-700d
▪ Color cards: Eibunsya Insatsujyo Co Ltd. 545, NAKANO, Midori-Ku, SAGAMIHARASHIKANAGAWA, 220-0207 JAPAN 000-0000 JAPAN
▪ Mixing beakers: Hauschild Engineering, Becher PP30-60 mL, 39231090
▪ Stirrer: IKA-Werke, Eurostar P1 Powercontrol-visk
▪ Heating plate: Heidolph MR3004 Safety plate
▪ Heating plate: 2Mag Stirring hotplate 15
▪ Balance: Mettler Toledo PM16, Mettler Toledo XP105M

### Preparation of the Coat Solution:

400.0 g deionized water was placed in a beaker (tara/empty=266.0g). The previously weighed Parteck COAT (actual=100.0g) was added and stirred in cold (450 rpm) within 2 minutes and 50 seconds. The mixture was then heated (Set:105°C) while stirring (450 rpm) until the PVA was completely dissolved; time from turning on the heater until Coat was dissolved took about 30 minutes, at 86°C solution temperature. The heater was then turned off and the solution was allowed to cool to room temperature for 180 minutes with stirring at 450 rpm. The solution was then weighed and the amount of 22.0g deionized water, which was evaporated during heating, was added again.

### Preparation of the film cast suspensions:

| **Weight share M100** | **5%** | **10%** | **15%** | **20%** | **30%** | **40%** |
|---|---|---|---|---|---|---|
| Parteck M100 [g] | 2,504 | 5,000 | 7,500 | 10,000 | 15,000 | 20,010 |
| Parteck Coat-solution. (wt=20%) [g] | 47,610 | 45,000 | 42,510 | 40,000 | 35,000 | 30,000 |
| Total [g] | 50,114 | 50,000 | 50,010 | 50,000 | 50,000 | 50,010 |

The needed amount of whitening pigment was weight in a Hauschild cup and then filled with the required amount of 20% Coating solution. The cup was then put in the Hauschild mixer for 2 minutes at 3000 rpm. The resulting suspension was left for 1 hour to solubilize Parteck M100. After 1 hour, each suspension was placed again in the Hauschild speedmixer for 2:00 minutes at 3000 rpm and after that applied directly to the color cards using a 400µm squeegee and color card puller. For each pigment, 3 coated color cards were prepared. The color cards coated in this way were then dried on a heating plate set at 50°C for 1 hour.

### Color card measurements

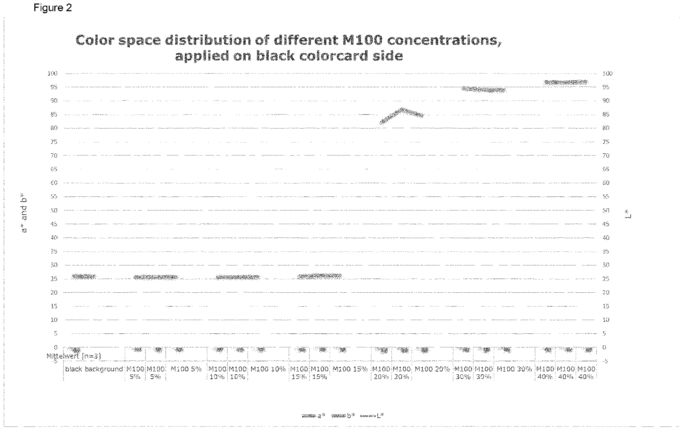

Color measurements were made using the Konica Minolta CM-700d spectrophotometer with a diskless MAV aperture. After the instrument was turned on, a zero calibration and a white calibration were first performed. The samples were each measured using the SCI and SCE methods. The black and the white section of a blanco color card were measured to generate reference values. For the actual measurement an area was defined for the color cards (4.5 cm up and down, 2.0 cm to the left and right of the center point) in which the measurements were conducted. Within this window, three points were measured in each of the white and black areas, and an average value was determined for the black and white sides using SpectraMagic NX software, which then described the color value for the black and white backgrounds of a color card. The results (see Fig. 2), given as L*a* b* values, showed that Mannitol performed best at concentration higher than wt.=30% in a Parteck Coat-solution.

### Example 2: Tablet coating

### Tableting raw tablet cores

### Equipment for tableting:

▪ Balance: Sartorius CAH1G-64FE-SCE
▪ Mixer: Free fall mixer, stainless-steel drum
▪ Sieves: 1,0 & 0,25 mm
▪ Tablet press: Rotary tablet press Fette 1200i with 11 mm domed round matrices.
▪ Hardness tester: Erweka Multicheck 5.1 (ERWEKA GmbH, Heusenstamm, Germany)
▪ Disintegration tester: Biomation Disi 4 (Biomation, Jugenheim, Germany)
▪ Tablet Friction tester: ERWEKA TA 420 (ERWEKA GmbH, Heusenstamm, Germany)
▪ IR-Balance: Mettler Halogen Moisture Analyzer HC103 (Mettler Toledo GmbH, Gießen, Germany)

19,6 kg of Parteck^{®} DPI (98%) and 0,1 kg Iron oxide (0,5%) were sieved with a 1,0 mm sieve and weight in a stainless-steel mixing drum. It was mixed with a free fall mixer for 10 minutes at 42 rpm. 0,3 kg of Parteck^{®} LUB MST (1,5%) was then sieved with a 250 µm sieve, added to the other components and mixed again for 10 minutes in a free fall mixer with 42 rpm. This procedure was done two times to receive 40 kg of fine powdered, red tableting mixture in total.

The mixture was then filled in the stirring blade feeder shoe of the tablet press at 30 rpm mixing speed. The tableting was done with a speed of 74 [tablets/h x 1.000] with a precompression force of 1,1 kN and a compressing force of 10,7 kN. Filling depth was 8,27 mm, height of headspace was 4,5 mm for precompression and 2,91 mm for main compression. The process led to a total amount of 37,5 kg tablets after 2,5 hours process time.

The tableting resulted in tablets with a rose colored, smooth and shiny surface. Small red dots were visible on the surface, due to the contained iron oxide. Tablet height was 5,1 mm and width were 11,1 mm. The tablets had an average weight of 498,55 mg and a hardness of 266 N, disintegration was achieved in an average of 325 seconds.

### Coating of raw tablet cores

### Equipment:

▪ Coater: Freund-Vector LDCS (Freund-Vector Corporation, Marion, United States)
▪ Overhead stirrer: IKA-Werke Eurostar P1 Powercontrol-visk; Janke&Kunkel IKV Werke RW 20 DZM (IKA-Werke GmbH & CO. KG, Staufen, Germany) 79219 Staufen / Deutschland
▪ Heating plate: Heidolph MR3004 Safety plate (Heidolph Instruments GmbH & Co. KG Walpersdorfer, Germany)
▪ Ultra Turrax: IKA-Werke, Ultra turraxT 25 Basic (IKA-Werke GmbH & CO. KG, Staufen, Germany)
▪ Peristaltic pump: Heidolph Pumpdrive 5201 (Heidolph Instruments GmbH & Co. KG, Walpersdorfer, Germany)

450.00 g deionized water was poured in a beaker glass. 45,00 g Parteck^{®} COAT added to the water and was stirred in cold at 450 rpm within 22 seconds. Then the water was heated (Set:105°C) until the PVA was completely dissolved (Time: 25 minutes and 11 sec.) at 450 rpm. The coating solution was then cooled to room temperature for 180 minutes under stirring at 310 rpm. The solution was stored in the refrigerator overnight and brought back to room temperature in 60 minutes under stirring (200 rpm) the following day. Afterwards, the coating solution was weighed to determine the evaporized amount of H2O during the heating step (35,7 g). In the next step 29,18 g triethyl citrate was added under stirring at 200 rpm until completely dissolved. The solution was then divided into 2 beakers of approximately 200-300 ml each. In one of the beakers, the talcum (actual=15.00 g) was added and homogenized using an ultra-turrax mixer. In the other beaker, the mannitol M 100 (actual = 60.00 g) was added and homogenized via ultra-turrax mixer. Afterwards, both suspensions were poured together again, and the two beakers were rinsed with 35,7 g of the correspondingly missing deionized water (which was evaporated by heating to dissolve the PVA). The obtained 600,0 g coating solution then used for the actual coating process of the red tablet cores prepared previously. 1,1 kg of red tablet cores were added to the 2,5l coating drum of the Freund-Vector LDCS coater. The coating suspension was placed on magnetic stirrer and a stirring bar was added to the suspension. The stirrer itself was placed on a balance and the balance was set to tara zero. The weight loss of coating suspension during the coating process was monitored. The stirring speed was set to 800 rpm, which was later (process time: 34 min) decreased to 600 rm. The suspension was stirred until the coating process was finished. Drum rotation was started at 10 rpm in tip modus. The inlet temperature heater was started. While the coating chamber was heating up to the target inlet temperature of 75°C, the spray rate of the peristaltic pump was calibrated. The calibration was done by starting the pump at 3 different rpm rates for 60 seconds. The coating suspension dripping out of the nozzle within each rate was poured into a beaker and the amount was weight. The following rates were determinized:

| | |
|---|---|
| 5rpm | 5,53 g |
| 8rpm | 8,72 g |
| 10rpm | 11,1 g |

After the target inlet temperature was reached, the coating drum was set to 10 rpm and the coating process was started with a pump rate of 6. The Coating process ran until the target amount of 403,0 g of coating solution was sprayed onto the raw tablet cores.

At 13.2 min, the drum speed was increased to 15 because the tablet bed did not flow continuously in the drum but kept slipping in a pulsating manner. The drum speed was further increased to 20 rpm at 14.7 min and even 25 rpm at 20 minutes coating process time. The spraying rate was also increased during the coating process since the outlet temperature started to climb. After the targeted amount of coating solution was applied to the tablet cores, the peristaltic pump was stopped, and the coating modus was remained for 2 minutes including inlet air heating and drum rotation. Within this time outlet temperature climbed to 43,1°C. After the 2 minutes have passed, the coating process was stopped by setting the inlet temperature to 30°C and stopping the drum rotation. The coater was opened for a couple of minutes to support colling down the process room to 30°C. After the inlet air reached the set temperature, the coater door was closed again and the tablets were left in the coater for 2 hours to dry at 30°C inlet temperature.

### Applied coating parameters:

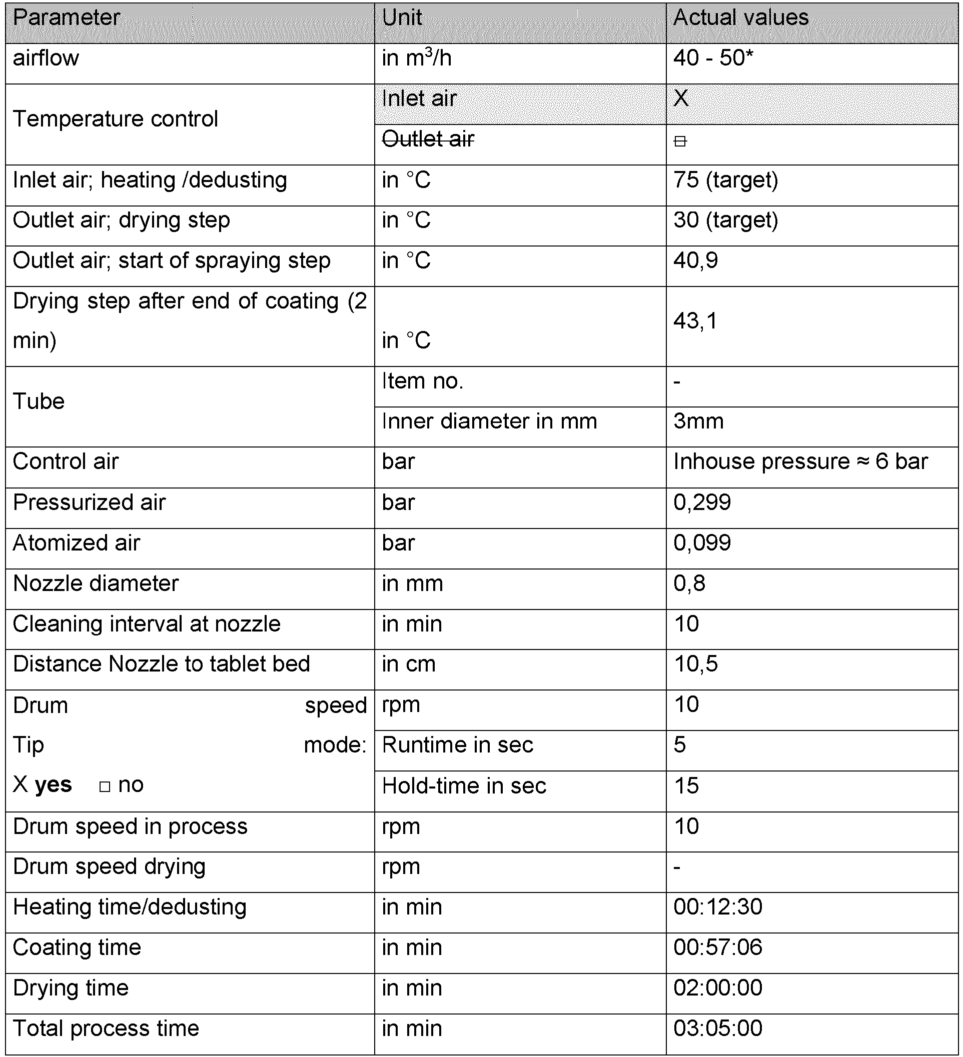

### IPC and 1 day tablet properties

The obtained tablets were tested for disintegration time, friability, loss of weight, hardness, average tablet weigh, height and diameter. The test values included IPC and day 1 values.

The obtained values were like followed:

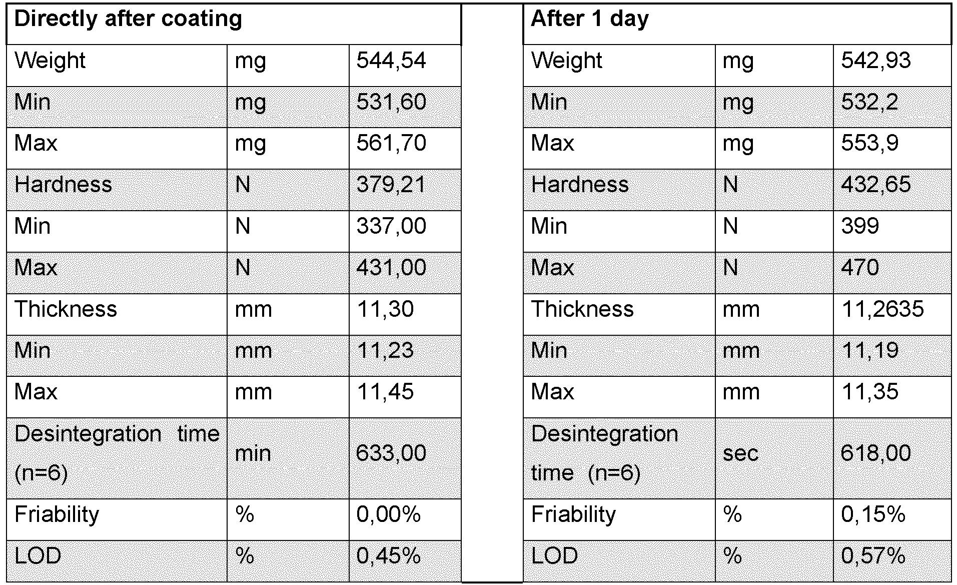

### Analysis of the tablets - hardness, weight, height and diameter

| | | |
|---|---|---|
| Device designation: | | Erweka Multicheck 5.1 (ERWEKA GmbH, Heusenstamm, Germany) |
| Tablets: | | n = 20 |
| Hardness: | | no requirements according to USP / Ph. Eur. |
| Tablet weight: | | Maximum permissible deviation from the average mass in % (non-coated and film-coated tablets, Ph. Eur.): |
| Average mass: | | ≤ 80 mg → 10% |
| | | 80 - ≤ 250 mg → 7,5 % |
| | | ≥ 251 mg → 5 % |
| | | Weigh individually 20 units taken at random or, for single-dose preparations supplied in individual packaging, the contents of 20 units, and determine the average mass. Unless specified in the individual monograph, not more than 2 of the individual masses |
| | deviate from the average mass by more than the percentage deviation shown in Table 2.9.5.-1 and none deviate by more than twice that percentage. | |
| Height: | no requirements according to USP / Ph. Eur. | |
| Diameter: | no requirements according to USP / Ph. Eur. | |
| Disintegration time | | |
| Device designation: | disi4 (Biomation, Jugenheim, Germany) | |
| Conditions: | Tablets: | n = 6 |
| | Media: | Deionized water |
| | Amount: | 800 mL |
| | Temperature: | 36 - 38 °C |
| | Frequency: | 28 - 32 1/min |
| | Hub: | 53 - 57 mm |
| | Others: | measurement with Disks |
| | Specification: | < 15 min (non-coated tablets) |

### Loss on Dry - IR balance

| | |
|---|---|
| Device designation: | Mettler PM 400; Mettler LP16 (Mettler Toledo GmbH, Gießen, Germany) |
| Sample weight: | 10 - 15g |
| Temperature: | 105 °C |
| Method: | 0 - 100% |
| Aluminum shell: | Ø = 95mm, #MLB-A01, Kern & Sohn GmbH, Balingen, Germany) |

### Color measurements of coated tablets

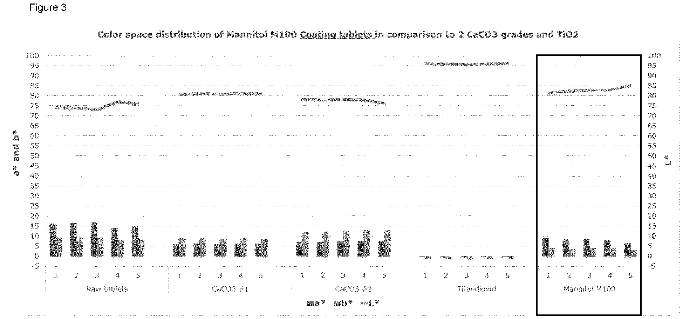

The coated tablets were measured with a Konica Minolta MC-700d spectrophotometer. Color measurements were made using the Konica Minolta CM-700d spectrophotometer with the MAV aperture without a disk. After the instrument was turned on, a zero calibration and a white calibration were first performed. The samples were each measured using the SCI and SCE methods (see Fig. 3).

Compared to CaCO3 and TiO2, Mannitol showed promising results when used as a coating pigment. The a and b values linked to the red and yellow coloring of the core tablets were successfully reduced. The reflection of light was increased confirmed by elevated L values indicating a strong reflection of the incoming light.

## Claims

1. A coating solution, comprising
- a polyol,
- a polymer,
- a solvent, and,
- optionally, a plasticizer;
wherein the polyol is mannitol or xylitol and the polymer is an aqueous soluble polymer or a cellulose based polymer.

2. The coating solution of claim 1, wherein the aqueous soluble polymer is selected from the group consisting of polyvinyl alcohol (PVA), copovidone vinylpyrrolidone-vinyl acetate copolymers (PVP-VA), polyvinyl pyrrolidone, methacrylic acid copolymers, and shellac; and/or wherein the cellulose based polymer is selected from the group comprising methylcellulose, hydroxypropyl cellulose (HPC), hydroxypropyl methylcellulose (HPMC or hypromellose), hydroxyethyl cellulose (HEC), hydroxyethyl methyl cellulose (HEMC), ethylcellulose (EC), and cellulose acetate phthalate.

3. The coating solution of any of claims 1 to 2, wherein the polyol is mannitol and the polymer is PVA, the w/w% ratio of mannitol to PVA being preferably between about 40 : 60, preferably,50 : 50 and more preferably 85:15.

4. The coating solution of any of claims 1 to 3, wherein the coating solution comprises mannitol at a w/w% concentration between 5 and 40%, preferably between 7.5% and 15%.

5. The coating solution of any of claims 1 to 4, further comprising an anti-tacking agent, wherein the anti-tacking agent is preferably selected from the group consisting of talcum, magnesium stearate, microcrystalline cellulose, glyceryl monostearate, stearic acid, kaolin and colloidal silicon dioxide.

6. The coating solution of any of claims 1 to 5, wherein the plasticizer is selected from the group comprising triethylcitrate, triacetin, glycerol, and polyethylene glycols of molecular weights between 600 and 6000 Da, preferably between 1000 and 3000 Da.

7. The coating solution of any of claims 1 to 6, wherein the solvent is a water-based solvent, an alcohol, a ketone, an ester or a chlorinated hydrocarbon or a mix thereof.

8. The coating solution of any of claims 1 to 7, wherein the color distribution values of the coating solution according to L*a* b* values of CIELAB color space are
L* > 90, preferably L* > 95,
-5 < a* < 10, preferably -2 < a* < 5, preferably -1 < a* < 1, more preferably a* = 0, and
-5 < b* < 10, preferably -2 < b* < 5, preferably -1 < b* < 1, more preferably b* = 0,
as measured by color card trials.

9. Use of the coating solution of any of claims 1 to 8, as a coating of a medicament, preferably a tablet.

10. A method for coating a medicament, comprising the steps of
a) mixing a solution comprising a polyol, a polymer, a solvent and optionally a plasticizer;
b) spraying the solution of step a) on a medicament;
c) allowing the evaporation of the solvent,
wherein the polyol is mannitol or xylitol and the polymer is an aqueous soluble polymer or a cellulose based polymer.

11. The method of claim 10, wherein the mannitol is added during the mixing step a) at a w/w% concentration between 10% and 30%, preferably between 20% and 30%.

12. The method of claim 10 or 11, wherein the polyol crystallizes on the medicament surface during step c).

13. The method of any of claims 10 to 12, wherein the solvent is a water-based solvent, an alcohol, a ketone, an ester or a chlorinated hydrocarbon or a mix thereof.

14. The method of claim 13, wherein a water-based solvent is used and a second solvent during step a) is added at a w/w% concentration of up to 30% of the water-based solvent in order to improve evaporation during step c).

15. Use of mannitol in a coating solution or of a coating solution comprising mannitol, preferably according to any of claims 1 to 8, for imparting a white color to a medicament, preferably a tablet.
